(19) Europäisches Patentamt
European Patent Office
Office européen des brevets

(11) EP 1 843 770 B1

(12) **EUROPEAN PATENT SPECIFICATION**

(45) Date of publication and mention of the grant of the patent:
**18.06.2008 Bulletin 2008/25**

(21) Application number: **06802484.3**

(22) Date of filing: **29.08.2006**

(51) Int Cl.:
***A61K 31/4985*** *(2006.01)*

(86) International application number:
**PCT/US2006/033541**

(87) International publication number:
**WO 2007/027612 (08.03.2007 Gazette 2007/10)**

# (54) SOLID PARTICULATE TADALAFIL HAVING A BIMODAL PARTICLE SIZE DISTRIBUTION

FESTES, TEILCHENFÖRMIGES TADALAFIL MIT BIMODALER TEILCHENGRÖSSENVERTEILUNG

TADALAFIL A PARTICULES SOLIDES A DISTRIBUTION GRANULOMETRIQUE BIMODALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.08.2005 US 712589 P**

(43) Date of publication of application:
**17.10.2007 Bulletin 2007/42**

(73) Proprietor: **Teva Pharmaceutical Industries Ltd**
**49131 Petah Tiqva (IL)**

(72) Inventors:
- **ARONHIME, Judith**
**76217 Rehovot (IL)**
- **SAMBURSKI, Guy**
**42930 Ganot-Hadar (IL)**
- **OVADYA, Yhoshoa**
**Migdal Haemek (IL)**

(74) Representative: **Gallagher, Kirk James et al**
**D Young & Co**
**120 Holborn**
**London EC1N 2DY (GB)**

(56) References cited:
**EP-A1- 0 967 214**
**WO-A-02/094219**
**US-B1- 6 821 975**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 843 770 B1

## Description

### FIELD OF THE INVENTION

The field of the invention relates to solid particulate tadalafil having a bimodal particle size distribution.

### BACKGROUND OF THE INVENTION

Tadalafil is a potent and selective inhibitor of the cyclic guanosine monophosphate (cGMP) - specific phosphodiesterase enzyme, PDE5. The inhibition of PDE5 increases the amount of cGMP, resulting in smooth muscle relaxation and increased blood flow. Tadalafil is therefore currently used in the treatment of male erectile dysfunction. Tadalafil is reported in the PDR to be a crystalline solid that is practically insoluble in water. The particle size of a poorly water soluble drug is reportedly influenced by the size of the particle of the drug. United States Patent No. 6,821,975 claims a free drug particulate form of a compound having a formula

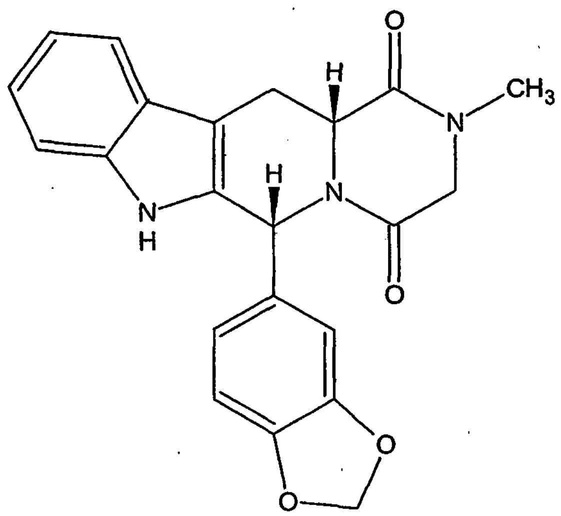

comprising particles of the compound wherein at least 90% of the particles have a particle size of less than about 40 microns (40μ).

Particle size distribution ("PSD") of a poorly water soluble drug such as tadalafil may greatly affect its bioavailability. Yet, when a active pharmaceutical ingredient is obtained with a relatively low particle size, the process of reducing particle size, such as milling, adversely affects the properties of the powder. For example, a great amount of highly fine solids can be produced by milling which may adversely affect the flow properties of the powder. There is a need in the art for tadalafil with a desirable PSD, which has otherwise good properties (such as flow properties).

### SUMMARY OF THE INVENTION

In one embodiment, the present invention provides solid particulate tadalafil having a bimodal size distribution.

In one embodiment, the present invention provides solid particulate tadalafil having at least one of the following particle size distribution as determined by volume by laser-diffraction method:

a) $41\mu < d(0.9) \leq 81\mu$;

b) $41\mu < d(0.9)$;

c) $41\mu < d(0.7) \leq 81\mu$;

d) $45\mu < d(0.9) \leq 70\mu$;

e) $60\mu \leq d(0.5) \leq 80\mu$;

f) 5μ ≤d(0.5) ≤15μ;

g) 10μ ≤d(0.5) ≤15μ;

h) 5μ ≤d(0.5) ≤10μ;

i) 1μ ≤d(0.1) ≤2μ.

In one embodiment, the present invention provides a process of preparing solid particulate tadalafil of any of the above embodiments comprising combining solid particulate tadalafil having at least two different particle size distributions.

Also provided are pharmaceutical compositions and use thereof.

## BRIEF DESCRIPTION OF THE FIGURES

Figure 1 shows a representative particle size distribution for a solid particulate tadalafil within the scope of the invention.

## DETAILED DESCRIPTION OF THE INVENTION

One embodiment of the present invention includes solid particulate tadalafil having a bimodal particle size distribution. Solid tadalafil in powder or particulate granular form comprised of a plurality of discrete particles, or individual units of mass, of tadalafil that range in nominal size from a few to a few hundred microns, μ ($10^{-6}$ m). Bimodal distribution refers to a distribution of particles where two maxima are present.

Since only a fraction of the tadalafil has to be reduced in size for bimodal distribution, the final product has fewer particles that have undergone an excessive reduction in particle size; these particles are fine solids which adversely affect the flow properties of a powder. Furthermore, bimodal particle size distribution generally has advantages such as good bulk properties, good mixing properties, and good dissolution profiles.

Frequently it is inconvenient or impractical to compare samples of particulate tadalafil on the basis of the entire cumulative PSD (Particle Size Distribution) or its first derivative. In most cases relevant to the invention, it is possible to compare samples of solid particulate tadalafil on the basis of individual points on the cumulative distribution curve, represented as d(0.*X*) = *Y* (where *X* and *Y* are Arabic numerals), each "d" describing an individual point on the cumulative PSD curve. The number "*X*" represents the percentage (number, volume, or weight) of particles in the population having a nominal size up to and including "Y". That is, d(0.9) = 41μ is the distribution such that 90% of the particles have a volume of this value or less. When 41μ < d(0.9) ≤81 μ, it means that 90% of the particles have a volume below a value in this range. For example, if such value is 60 μ, 90% of the particles have a volume of zero to 60 μ.

In one embodiment, the invention provides solid particulate tadalafil having at least one of the following PSD (which are bimodal unless used as starting material):

A1) 41μ < d(0.9) ≤81μ;

A2) 41μ < d(0.9);

A3) 41μ<d(0.7) ≤81μ;

A4) 45μ < d(0.9) ≤70μ;

B1) 5μ ≤d(0.5) ≤15μ;

B2) 10μ ≤d(0.5) ≤15μ;

B3) 5μ ≤d(0.5) ≤10μ;

C) 1μ ≤d(0.1) ≤2μ.

The present invention provides the following combinations of size distributions: A1 alone; A2 alone; A3 alone; A4 alone;

A5 alone; B1 alone; B2 alone; B3 alone; C alone; combination of A1 and B1; combination af A1 and B2; combination of A1 and B3; combination of A1 and C; combination of A1, B1 and C; combination of A1, B2, and C; combination of A1, B3., and C; combination of A2 and B1; combination of A2 and B2; combination of A2 and B3; combination of A2 and C; combination of A2, B1, and C; combination of A2, B2, and C; combination of A2, B3, and C; combination of A3 and B1; combination of A3 and B2; combination of A3 and B3; combination of A3 and C; combination of A3, B1, and C; combination of A3, B2, and C; combination of A3, B3, and C; combination of A4 and B1; combination of A4 and B2; combination of A4 and B3; combination of A4 and C; combination of A4, B1, and C; combination of A4, B2, and C; combination of A4, B3, and C; combination of A5 and B1; combination of A5 and B2; combination of A5 and B3; combination of A5 and C; combination of A5, B1, and C; combination of A5, B2, and C; combination of A5, B3, and C; combination of B1 and C; combination of B2 and C; and combination of B3 and C.

Preferred bimodal PSDs include 41μ < d(0.9) ≤80μ; combination of 41μ < d(0.9) ≤ 81μ and 5μ ≤ d(0.5) ≤ 15μ; 41μ < d(0.7) ≤ 81μ; combination of 41μ < d(0.9) ≤ 81μ, 5μ ≤ d (0.5) ≤ 15μ and 1μ ≤ d(0.1) ≤2μ.

In another embodiment, the present invention provides solid particulate tadalafil wherein 1μ ≤d(0.1) ≤2μ; 5μ ≤d(0.5) ≤10μ; and 41μ < d(0.9) ≤80μ.

These PSDs are bimodal. For example, when the PSD is A1, two maxima are obtained in the PSD in the range of zero and a value between 41 μ and 81 μ. In one embodiment a bimodal PSD is obtained by combining any of A1-C with each other,

where the starting material is not bimodal but the end product of the combination is bimodal.

Any of the above solid particulate tadalafil with the bimodal size distribution can be made by combining a first, large particle size solid particulate tadalafil with a second small particle size solid particulate tadalafil. The size of the particles can be adjusted depending on the PSD desired. The combining can be by any powder-combining means known in the art provided that the energy input from the combining equipment to the first and second particulate tadalafil does not cause substantial particle attrition.

The small and large particle size solid particulate tadalafil can be prepared by conventional techniques. For example, after being characterized for size in its raw state, the tadalafil may is milled, for example using a pin mill under suitable conditions of mill rotation rate and feed rate, to bring the particle size value within a desirable range. The efficiency of the milling may be monitored by sampling. If a first pass through the mill fails to produce the required size distribution, then one or more further passes are effected. Other methods are readily available, including, a variety of milling techniques, such as hammer or fluid energy mills.

In one embodiment, solid particulate tadalafil having a bimodal PSD of 40μ (d(0.9) ≤ 41μ), such as 41μ < d (0.9) ≤ 81μ and/or 45μ ≤ d(0.9) ≤70μ is prepared by combining a first, large particle size solid particulate tadalafil, wherein d(0.1) ~ 10μ and d(0.9) - 120μ, with a second, small particle size solid particulate tadalafil, wherein at least about 98 volume-% of the particles of the second, small particle size tadalafil have a nominal particle size of about 15μ or less. In one embodiment, the small particle size solid particulate tadalafil is d(0.9) < 10 μ and the large particle size solid particulate tadalafil is d(0.9)=150μ and 10μ ≤ d(0.1).

The individual particles of the solid particulate tadalafil can be regular-shaped, or they can have an irregular shape. In most embodiments of the invention they are irregular shaped. When the particles have an irregular shape, nominal size of a particle refers to the dimension of the so-called equivalent sphere, a concept well-known in the field of particle size analysis.

The particles of which the solid particulate tadalafil of the invention is comprised are discrete particles that, in particular embodiments, can be more or less tightly bound agglomerates that to not dissociate when subjected to low intensity ultrasonification of short duration.

The individual particles of a sample or aliquot of the solid particulate tadalafil of the invention are not of uniform size. Rather, a sample or aliquot of a solid particulate tadalafil of the invention is comprised of particles of different sizes that can be size-classified or distributed in an array of discrete, adjacent intervals of particle size. If the size of the intervals is small enough, the array of particle sized approaches a continuum of particle sizes. This collection of discrete particle size intervals together with their population is referred to as the particle size distribution (PSD).

Measurement and characterization of particle size distributions is well-known in the art. Particle size distribution can be represented graphically as shown in Figure 1, which depicts the particle size distribution of a solid particulate tadalafil of the invention. Curve A is the so-called cumulative distribution. The ordinate represents the fraction (number, volume, or weight fraction, preferably volume fraction) of particles in a population having a nominal particle size up to the size indicated on the coordinate point on the abscissa. Curve B depicts the first derivative of the cumulative particle size distribution and is frequently referred to as the "discrete distribution". The first derivative of the cumulative distribution - or the discrete distribution - exhibits maxima at points corresponding to inflection points in the cumulative distribution curve. As shown in Figure 1, the first derivative of the cumulative PSD of the solid particulate tadalafil of the invention exhibits two maxima and the PSD is accordingly denoted "bimodal". A bimodal PSD is one distinguishing characteristic of the solid particulate tadalafil of the invention.

Whether d(0.*X*) depicts a number, volume, or weight percentage depends on the method used to determine

the PSD. Several methods of determining PSD and the type of percentage associated therewith are known in the art. When PSD is determined by the well-known laser-diffraction method, performed according to the method described below, d(0.*X*) depicts a volume average. The skilled artisan knows that the results of PSD determination by one technique can be correlated with that from another technique on an empirical basis by routine experimentation.

The PSD of the solid particulate tadalafil of the invention is preferably determined using the well-known laser diffraction method. The laser-diffraction method (low angle laser light scattering) is sensitive to the volume of a particle and provides a volume-average particle size, which is equivalent to the weight-average particle size if the density is constant. In some instances, particle size determination by laser diffraction employs the so-called Fraunhofer approximation - based on projected area - which includes the assumptions that particles size is not smaller than the wavelength of light constant scattering efficiency, and particle opacity. The "Mastersizer S" from Malvern Instruments equipped with a small cell dispersion unit is an example of a laser-diffraction particle size analyzer useful in determining the PSD of solid particulate tadalafil of the present invention. Any laser diffraction method that would give an accurate result can be used.

Samples for measurement using the Mastersizer S can be prepared by combining the sample with a media such as a silicone fluid having a viscosity at about 25° C of about 10 cst. Dow Corning 200(R) F-10 and Silicaid® F-10 are examples of diluents useful in particle size measurement. The combination of sample and diluent (e.g. F-10 silicone fluid) is mixed by vortex mixing for about 20 seconds followed by sonification for about 5 seconds. Excessive sonification should be avoided to avoid break-up of hard agglomerates.

Another embodiment of the invention provides pharmaceutical compositions that contain solid particulate tadalafil having a bimodal PSD. The pharmaceutical compositions may contain the following combinations of size distributions: A1 alone; A2 alone; A3 alone; A4 alone; A5 alone; B1 alone; B2 alone; B3 alone; C alone; combination of A1 and B1; combination of A1 and B2; combination of A1 and B3; combination of A1 and C; combination of A1, B1 and C; combination of A1, B2, and C; combination of A1, B3, and C; combination of A2 and B1; combination of A2 and B2; combination of A2 and B3; combination of A2 and C; combination of A2, B1, and C; combination of A2, B2, and C; combination of A2, B3, and C; combination of A3 and B1; combination of A3 and B2; combination of A3 and B3; combination of A3 and C; combination of A3, B1, and C; combination of A3, B2, and C; combination of A3, B3, and C; combination of A4 and B1; combination of A4 and B2; combination of A4 and B3; combination of A4 and C; combination of A4, B1, and C; combination of A4, B2, and C; combination of A4, B3, and C; combination of A5 and B1; combination of A5 and B2; combination ofA5 and B3; combination of A5 and C; combination of A5, B1, and C; combination of A5, B2, and C; combination of A5, B3, and C; combination of B1 and C; combination of B2 and C; and combination of B3 and C. In one embodiment, at least 10 volume-% of the particles have a particle size greater than 40μ (d(0.9) ≥41μ), such as 41μ < d(0.9) ≤81μ and/or 45μ < d(0.9) ≤70μ.

The pharmaceutical compositions can be prepared by combining tadalafil having a bimodal PSD with a pharmaceutically acceptable excipient. In one embodiment, large particle size solid particulate tadalafil, wherein d(0.1) ~ 10μ and d(0.9) ~ 120μ, is combined with a second, small particle size solid particulate tadalafil, wherein at least about 98 volume-% of the particles of the second, small particle size tadalafil have a nominal particle size of about 15μ or less; this mixture is admixed with at least one pharmaceutically acceptable excipient. In one embodiment, the small particle size solid particulate tadalafil is d(0.9) < 10 μ and the large particle size solid particulate tadalafil is d(0.9)=150μ and 10μ ≤d(0.1).

In addition to the active ingredient(s), the pharmaceutical compositions of the invention contain one or more excipients. Excipients are added to the formulation for a variety of purposes.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel®), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®), potassium chloride, powdered cellulose, sodium chloride, sorbitol and talc.

Solid pharmaceutical compositions that are compacted into a dosage form, such as a tablet, may include excipients whose functions include helping to bind the active ingredient-and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel®), hydroxypropyl methyl cellulose (e.g. Methocel®), liquid glucose, magnesium aluminum silicate, maltodextrin, methylcellulose, polymethacrylates, povidone (e.g. Kollidon®, Plasdone®, pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol®, Primellose®), colloidal silicon dioxide, croscarmellose sodium, crospovidone (e.g. Kollidon®, Polyplasdone®), guar gum, magnesium aluminum silicate, methyl cellulose, microcrystalline cellulose, polacrilin potassium, powdered cellulose, pregelatinized starch, sodium alginate, sodium starch glycolate (e.g. Explotab®) and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl furmarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid and liquid compositions (suspensions or emulsions) may also be dyed using any pharmaceutically acceptable colorant to improve their appearance and/or facilitate, patient identification of the product and unit dosage level.

In liquid pharmaceutical compositions of the present invention, solid particulate tadalafil having a bimodal particle size distribution and any other solid excipients are suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.

Preservatives and chelating agents such as alcohol, sodium benzoate, butylated hydroxyl toluene, butylated hydroxyanisole and ethylenediamine tetraacetic acid may be added at levels safe for ingestion to improve storage stability.

According to the invention, a liquid composition may also contain a buffer such as gluconic acid, lactic acid, citric acid or acetic acid, sodium gluconate, sodium lactate, sodium citrate or sodium acetate. Selection of excipients and the amounts used may be readily determined by the formulation scientist based upon experience and consideration of standard procedures and reference works in the field.

The solid compositions of the invention include powders, granulates, aggregates and compacted compositions. Solid oral dosage forms are particularly preferred. Although the most suitable administration in any given case will depend on the nature and severity of the condition being treated, the most preferred route of the invention is oral. The dosages may be conveniently presented in unit dosage form and prepared by any of the methods well-known in the pharmaceutical arts.

Dosage forms include solid dosage forms like tablets, powders, capsules, suppositories, sachets, troches and losenges, as well as liquid syrups, suspensions and elixirs.

The dosage form of the invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

The invention in certain of its embodiments is illustrated with the following examples.

## EXAMPLES

Particle size and PSD was measured using a Malvern "Mastersizer S" (Malvern Instruments) fitted with a small cell dispersion unit, digital dispersion controller, and 300 RF lens (0.05μ to 900 μ). Samples were dispersed in a 10 cst F-10 silicone fluid by vortex mixing (10 sec.) followed by sonification (5 sec.). Samples were recirculated at circa 3000 rpm recirculator speed. Output was presented in 3$$D mode (Fraunhofer approximation).

The large particle size particulate tadalafil used in the examples had d(0.1)~ 10μ and d(0.9) ~ 120μ and ca. 53 % of the particles had d < 45μ.

The small particle size particulate tadalafil used in the examples had a maximum particle size of about 15 μ. Alternatively, a small particle size particulate tadalafil in which 99.96% of the particles have d < 45 μm can be used.

### EXAMPLE 1

Two grams of a solid particulate tadalafil having a bimodal particle size distribution according to the invention were prepared by combining 0.38 g of large particle size tadalafil and 1.62 g of small particle size tadalafil.

The amount of large particle size particulate tadalafil (x) was calculated as follows, where d = d(0.9) =40

$$\frac{52.6 \bullet x + 100 \bullet (2 - x)}{2} = 90$$

$$47.4 \bullet x - 198 = 180$$

$$47.4 \bullet x = 18$$

$$x = 0.38 gr$$

The bimodal particles size of the product had;
d(0.1) = 1.3-1.5 μm
d(0.5) = 5-8 μm
d(0.9) = 47-61 μm

### EXAMPLE 2

Two grams of a solid particulate tadalafil according to the invention were prepared by combining 0.42 g of large particle size tadalafil and 1.58 g of small particle size tadalafil. The amount of large particle size tadalafil (x) was calculated as follows,
where d = d(0.9) = 45.

$$\frac{56 \bullet x + 99 \bullet (2 - x)}{2} = 90$$

$$43 \bullet x - 198 = 180$$

$$43 \bullet x = 18$$

$$x = 0.42gr$$

The product had a bimodal PSD characterized by;
d(0.1) = 1.3-1.5 μm
d(0.5) = 5-9 μm
d(0.9) = 60-65 μm

<u>EXAMPLE 3</u>

Two grams of a solid particulate tadalafil having a bimodal PSD according to the invention were prepared by combining 0.54 g of large particle size tadalafil and 1.46 g of small particle size tadalafil. The amount of large particle size tadalafil (x) was calculated as follows; where d(0.9) = 60

$$\frac{66.7 \bullet x + 100 \bullet (2 - x)}{2} = 90$$

$$33.3 \bullet x - 198 = 180$$

$$33.3 \bullet x = 18$$

$$x = 0.54gr$$

The product had a bimodal PSD characterized by;
d(0.1) = 1.5-1.8 μm
d(0.5) = 10-15μm
d(0.9) = 60-80 μm

**Claims**

1. A solid particulate tadalafil having a bimodal size distribution.

2. The solid particulate tadalafil of claim 1 having at least one of the following particle size distribution as determined by volume by laser-diffraction method:

   a) 41μ<d(0.9)≤81μ;
   b) 41μ<d(0.9);
   c) 41μ < d(0.7) ≤81μ;
   d) 45μ < d(0.9) ≤70μ;
   e) 60μ ≤d(0.5) ≤80μ;
   f) 5μ ≤d(0.5) ≤15μ;
   g) 10μ ≤d(0.5) ≤15μ;
   h) 5μ ≤ d(0.5) ≤10μ;
   i) 1μ ≤d(0.1) ≤2μ.

**3.** The solid particulate tadalafil of claim 2 having a particle size distribution of 41μ < d(0.9) ≤ 81μ.

**4.** The solid particulate tadalafil of claim 2 having a particle size distribution of 41μ < d(0.9).

**5.** The solid particulate tadalafil of claim 2 having a particle size distribution of 41 μ < d(0.7) ≤81 μ.

**6.** The solid particulate tadalafil of claim 2 having a particle size distribution of 45μ < d(0.9) ≤70μ.

**7.** The solid particulate tadalafil of claim 2 having a particle size distribution of 5μ ≤d(0.5) ≤15μ.

**8.** The solid particulate tadalafil of claim 2 having a particle size distribution of 10μ ≤d(0.5) ≤15μ.

**9.** The solid particulate tadalafil of claim 2 having a particle size distribution of 5μ ≤d(0.5) ≤10μ.

**10.** The solid particulate tadalafil of claim 2 having a particle size distribution of 1μ ≤d(0.1) ≤2μ.

**11.** The solid particulate tadalafil of claim 2 having a particle size distribution of 60μ ≤(0.5) ≤80μ.

**12.** The solid particulate tadalafil of claim 2 having a particle size distribution of 41μ < d(0.9) ≤81μ and 5μ ≤d(0.5) ≤15μ.

**13.** The solid particulate tadalafil of claim 2 having a particle size distribution of 41μ < d(0.9) ≤81μ, 5μ ≤d(0.5) ≤15μ and 1μ ≤d(0.1) ≤2μ.

**14.** The solid particulate tadalafil of claim 2 having a particle size distribution of 41μ < d(0.9) ≤81μ, 5μ ≤d(0.5) ≤10μ and 1μ ≤d(0.1) ≤2μ.

**15.** The solid particulate tadalafil of claim 2 having a particle size distribution of 60μ ≤d(0.9) ≤80μ, 10μ ≤d(0.5) ≤15μ and 1μ ≤d(0.1) ≤2μ.

**16.** The solid particulate tadalafil of claim 2 having a particle size distribution of 60μ ≤d(0.9) ≤80μ, 5μ ≤d(0.5) ≤10μ and 1μ ≤d(0.1) ≤2μ.

**17.** The solid particulate tadalafil of claim 2 having a particle size distribution of 41μ < d(0.9) ≤81μ, 10μ ≤d(0.5) ≤15μ and 1μ ≤d(0.1) ≤2μ.

**18.** The solid particulate tadalafil of claim 2 having a particle size distribution of 41μ <d(0.9) ≤81μ, 5μ ≤d(0.5) ≤10μ and 1μ ≤d(0.1) ≤2μ.

**19.** A process of preparing solid particulate tadalafil of any of the preceding claims comprising combining at least two samples of solid particulate tadalafil having at different particle size distribution.

**20.** A process of preparing solid particulate tadalafil according to claim 19, wherein a first tadalafil with a PSD of d(0.1) ~ 10μ and d(0.9) ~ 120μ is combined with a second tadalafil having at least about 98 volume-% of the particles with a nominal particle size of about 15μ or less.

**21.** The process of claim 20, wherein the small particle size solid particulate tadalafil is d(0.9) < 10 μ and the large particle size solid particulate tadalafil is d(0.9)=150μ and 10μ ≤d(0.1).

**22.** A pharmaceutical composition comprising solid particulate tadalafil according to any of claims 1-18 and a pharmaceutically acceptable excipient.

**23.** A process for preparing a pharmaceutical composition according to claim 22 comprising combining the solid particulate tadalafil according to any of claims 1 to 18 with a pharmaceutically acceptable excipient.

**24.** Use of the solid particulate tadalafil according to any of claims 1 to 18 for the manufacture of a medicament for the treatment of sexual dysfunction.

**Patentansprüche**

1. Festes teilchenförmiges Tadalafil mit einer bimodalen Größenverteilung.

2. Festes teilchenförmiges Tadalafil nach Anspruch 1, welches wenigstens eine der nachfolgenden Teilchengrößenverteilungen, bestimmt auf Basis des Volumens mittels Laserdiffraktionsverfahren, hat:

   a) 41 μm < d (0,9) ≤ 81 μm,
   b) 41 μm < d (0,9),
   c) 41 μm < d (0,7) ≤ 81 μm,
   d) 45 μm < d (0,9) ≤ 70 μm,
   e) 60 μm ≤ d (0,5) ≤ 80 μm,
   f) 5 μm ≤ d (0,5) ≤15 μm,
   g) 10 μm ≤ d (0,5) ≤15 μm,
   h) 5 μm ≤d (0,5)≤10 μm,
   i) 1 μm≤d (0,1) ≤ 2 μm.

3. Festes teilchenförmiges Tadalafil nach Anspruch 2 mit einer Teilchengrößenverteilung von 41 μm < d (0,9)≤ 81 μm.

4. Festes teilchenförmiges Tadalafil nach Anspruch 2 mit einer Teilchengrößenverteilung von 41 μm < d (0,9).

5. Festes teilchenförmiges Tadalafil nach Anspruch 2 mit einer Teilchengrößenverteilung von 41 μm < d (0,7)≤ 81 μm.

6. Festes teilchenförmiges Tadalafil nach Anspruch 2 mit einer Teilchengrößenverteilung von 45 μm < d (0,9) ≤ 70 μm.

7. Festes teilchenförmiges Tadalafil nach Anspruch 2 mit einer Teilchengrößenverteilung von 5 μm ≤ d (0,5) ≤ 15 μm.

8. Festes teilchenförmiges Tadalafil nach Anspruch 2 mit einer Teilchengrößenverteilung von 10 μm ≤ d (0,5) ≤ 15 μm.

9. Festes teilchenförmiges Tadalafil nach Anspruch 2 mit einer Teilchengrößenverteilung von 5 μm ≤ d (0,5) ≤ 10 μm.

10. Festes teilchenförmiges Tadalafil nach Anspruch 2 mit einer Teilchengrößenverteilung von 1 μm ≤ d (0,1) ≤ 2 μm.

11. Festes teilchenförmiges Tadalafil nach Anspruch 2 mit einer Teilchengrößenverteilung von 60 μm ≤ d (0,5) ≤ 80 μm.

12. Festes teilchenförmiges Tadalafil nach Anspruch 2 mit einer Teilchengrößenverteilung von 41 μm < d (0,9) ≤ 81 μm und 5μm ≤ d (0.5) ≤15μm.

13. Festes teilchenförmiges Tadalafil nach Anspruch 2 mit einer Teilchengrößenverteilung von 41 μm < d (0,9) ≤ 81μm, 5 μm ≤d (0,5)≤15 μm und 1μm≤ d (0,1) ≤ 2μm.

14. Festes teilchenförmiges Tadalafil nach Anspruch 2 mit einer Teilchengrößenverteilung von 41 μm < d (0,9)≤ 81μm, 5μm ≤ d (0,5) ≤ 10 μm und 1μm ≤ d (0,1) ≤ 2 μm.

15. Festes teilchenförmiges Tadalafil nach Anspruch 2 mit einer Teilchengrößenverteilung von 60 μm ≤ d (0,9) ≤ 80 μm, 10 ≤ d (0.5) ≤15μm und 1μm ≤ d (0.1) ≤ 2μm.

16. Festes teilchenförmiges Tadalafil nach Anspruch 2 mit einer Teilchengrößenverteilung von 60 μm ≤ d (0,9) ≤ 80 μm, 5 μm ≤ d (0,5) ≤ 10 μm und 1 μm ≤ d (0,1) ≤ 2 μm.

17. Festes teilchenförmiges Tadalafil nach Anspruch 2 mit einer Teilchengrößenverteilung von 41 μm ≤ d (0,9) ≤ 81 μm, 10 μm ≤ d (0,5) ≤15 μm und 1 μm ≤ d (0,1) ≤ 2 μm.

18. Festes teilchenförmiges Tadalafil nach Anspruch 2 mit einer Teilchengrößenverteilung von 41 μm < d(0,9) ≤ 81 μm, 5 μm ≤ d (0,5) ≤ 10 μm und 1 μm ≤ d (0,1) ≤ 2 μm.

19. Verfahren zur Herstellung von festem teilchenförmigem Tadalafil nach einem der vorangegangenen Ansprüche, welches das Vereinigen wenigstens zweier Proben von festem teilchenförmigem Tadalafil mit unterschiedlicher

Teilchengrößenverteilung umfaßt.

**20.** Verfahren zur Herstellung von festem teilchenförmigem Tadalafil nach Anspruch 19, wobei ein erstes Tadalafil mit einer PSD von d (0,1) ~ 10 μm und d (0,9) ~ 120 μm mit einem zweiten Tadalafil, welches wenigstens etwa 98 Volumen-% an Teilchen mit einer nominellen Teilchengröße von etwa 15 μm oder weniger enthält, vereinigt wird.

**21.** Verfahren nach Anspruch 20, wobei das feste teilchenförmige Tadalafil mit kleiner Teilchengröße d (0,9) < 10 μm ist und das feste teilchenförmige Tadalafil mit großer Teilchengröße d (0,9) = 150 μm und 10 μm ≤ d (0,1) ist.

**22.** Pharmazeutische Zusammensetzung, welche festes teilchenförmiges Tadalafil nach einem der Ansprüche 1 bis 18 und einen pharmazeutisch verträglichen Hilfsstoff umfaßt.

**23.** Verfahren zur Herstellung einer pharmazeutischen Zusammensetzung nach Anspruch 22, welches das Vereinigen des festen teilchenförmigen Tadalafils nach einem der Ansprüche 1 bis 18 mit einem pharmazeutisch verträglichen Hilfsstoff umfaßt.

**24.** Verwendung des festen teilchenförmigen Tadalafils nach einem der Ansprüche 1 bis 18 für die Herstellung eines Medikaments zur Behandlung einer Sexualstörung.

**Revendications**

**1.** Du tadalafil sous forme de particules solides ayant une distribution granulométrique bimodale.

**2.** Du tadalafil sous forme de particules solides selon la revendication 1, ayant au moins une des suivantes déterminées en volume par un procédé à diffraction laser :

a) 41 μm < d(0,9) 81 μm ;
b) 41 μm < d(0,9) ;
c) 41 μm < d(0,7) 81 μm ;
d) 45 μm < d(0,9) 70 μm ;
e) 60 μm d(0,5) 80 μm ;
f) 5 μm d(0,5) 15 μm ;
g) 10 μm d(0,5) 15 μm ;
h) 5 μm d(0,5) 10 μm ;
i) 1 μm d(0,1) 2 μm.

**3.** Du tadalafil sous forme de particules solides selon la revendication 2, dont la distribution granulométrique est: 41 μm < d(0,9) 81 μm.

**4.** Du tadalafil sous forme de particules solides selon la revendication 2, dont la distribution granulométrique est: 41 μm < d(0,9).

**5.** Du tadalafil sous forme de particules solides selon la revendication 2, dont la distribution granulométrique est: 41 μm < d(0,7) 81 μm.

**6.** Du tadalafil sous forme de particules solides selon la revendication 2, dont la distribution granulométrique est: 45 μm<d(0,9) 70 μm.

**7.** Du tadalafil sous forme de particules solides selon la revendication 2, dont la distribution granulométrique est: 5 μm d(0,5) 15 μm.

**8.** Du tadalafil sous forme de particules solides selon la revendication 2, dont la distribution granulométrique est: 10 μm d(0,5) 15 μm.

**9.** Du tadalafil sous forme de particules solides selon la revendication 2, ayant une distribution granulométrique of 5μm d(0,5) 10 μm.

**10.** Du tadalafil sous forme de particules solides selon la revendication 2, avant une distribution granulométrique of 1 µm d(0,1) 2 µm.

**11.** Du tadalafil sous forme de particules solides selon la revendication 2, dont la distribution granulométrique est: 60 µm (0,5) 80 µm.

**12.** Du tadalafil sous forme de particules solides selon la revendication 2, dont la distribution granulométrique est: 41 µm < d(0.9) 81 µm et 5 µm d(0,5) 15 µm.

**13.** Du tadalafil sous forme de particules solides selon la revendication 2, dont la distribution granulométrique est: 41 µm < d(0,9) 81 µm, 5 µm d(0,5) 15 µm et 1 µm d(0,1) 2 µm

**14.** Du tadalafil sous forme de particules solides selon la revendication 2, dont la distribution granulométrique est: 41 µm < d(0,9) 81 µm, 5 µm d(0,5) 10 µm et 1 µm d(0,1) 2 µm

**15.** Du tadalafil sous forme de particules solides selon la revendication 2, dont la distribution granulométrique est: 60 µm d(0,9) 80 µm, 10 µm d(0,5) 15 µm et 1 µm d(0,1) 2 µm

**16.** Du tadalafil sous forme de particules solides selon la revendication 2, dont la distribution granulométrique est: 60 µm d(0,9) 80 µm, 5µm d(0,5) 10 µm et 1 µm d(0,1) 2 µm

**17.** Du tadalafil sous forme de particules solides selon la revendication 2, dont la distribution granulométrique est: 41 µm < d(0,9) 81 µm, 10 µm d(0,5) 15 µm et 1 µm d(0,1) 2 µm

**18.** Du tadalafil sous forme de particules solides selon la revendication 2 dont la distribution granulométrique est: 41 µm < d(0,9) 81 µm, de 5 µm d(0,5) 10 µm et de 1µm d(0,1) 2 µm

**19.** Un procédé de préparation de tadalafil sous forme de particules solides selon l'une quelconque des revendications précédentes, comprenant l'étape consistant à combiner au moins deux échantillons de tadalafil sous forme de particules solides ayant une distribution granulométrique différente.

**20.** Un procédé de préparation de tadalafil sous forme de particules solides selon la revendication 19, dans lequel un premier tadalafil dont la distribution granulométrique est: d(0,1)~ 10 µm et d(0,9) ~ 120 µm, est combiné à un deuxième tadalafil ayant au moins environ 98% en volume des particules dont la granulométrie nominale est d'environ 15 µm ou moins.

**21.** Le procédé selon la revendication 20, dans lequel les petites particules solides du tadalafil ont une granulométrie de d(0,9) < 10 µm et les plus grosses particules solides de tadalafil ont une granulométrie de d(0,9)=150µm et 10 µm d(0,1).

**22.** Une composition pharmaceutique comprenant le tadalafil sous forme de particules solides selon l'une quelconque des revendications 1 à 18 et un excipient acceptable sur le plan pharmaceutique.

**23.** Un procédé de préparation d'une composition pharmaceutique selon la revendication 22, comprenant l'étape consistant à combiner le tadalafil sous forme de particules solides selon l'une quelconque des revendications 1 à 18 avec un excipient acceptable sur le plan pharmaceutique.

**24.** Utilisation du tadalafil sous forme de particules solides selon l'une quelconque des revendications 1 à 18 pour la fabrication d'un médicament en vue du traitement de dysfonctionnement sexuel.

Figure 1

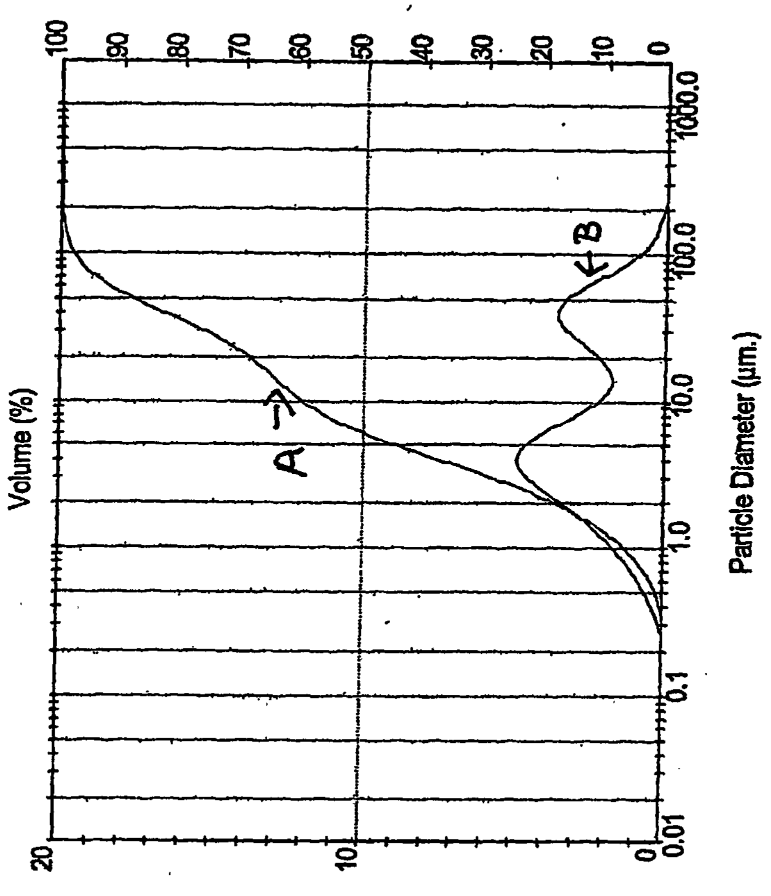
Volume (%)
Particle Diameter (µm.)
A
B
0
10
20
30
40
50
60
70
80
90
100
0.01
0.1
1.0
10.0
100.0
1000.0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6821975 B